**Europäisches Patentamt**

**European Patent Office**

**Office européen des brevets**

(19)

(11) Veröffentlichungsnummer: **0 458 939 B1**

(12) # EUROPÄISCHE PATENTSCHRIFT

(45) Veröffentlichungstag der Patentschrift :
**23.03.94 Patentblatt 94/12**

(51) Int. Cl.$^5$ : **A61K 37/54,** A61K 47/12, A61K 47/18, A61K 47/22

(21) Anmeldenummer : **91901285.6**

(22) Anmeldetag : **19.12.90**

(86) Internationale Anmeldenummer :
**PCT/EP90/02251**

(87) Internationale Veröffentlichungsnummer :
**WO 91/08766 27.06.91 Gazette 91/14**

(54) t-PA pro STABILISIERUNG.

Die Akte enthält technische Angaben, die nach dem Eingang der Anmeldung eingereicht wurden und die nicht in dieser Patentschrift enthalten sind.

(30) Priorität : **20.12.89 DE 3942143**

(43) Veröffentlichungstag der Anmeldung :
**04.12.91 Patentblatt 91/49**

(45) Bekanntmachung des Hinweises auf die Patenterteilung :
**23.03.94 Patentblatt 94/12**

(84) Benannte Vertragsstaaten :
**AT BE CH DE DK ES FR GB GR IT LI LU NL SE**

(56) Entgegenhaltungen :
**EP-A- 0 211 592**
**EP-A- 0 217 379**

(56) Entgegenhaltungen :
**EP-A- 0 297 294**
**WO-A-87/02673**
**WO-A-90/01333**
**WO-A-90/08557**

(73) Patentinhaber : **BOEHRINGER MANNHEIM GMBH**
**D-68298 Mannheim (DE)**

(72) Erfinder : **KOHNERT, Ulrich, Dr.**
**Heubachweg 6**
**D-8121 Habach (DE)**
Erfinder : **RUDOLPH, Rainer, Dr.**
**Färbergasse 19**
**D-8120 Weilheim (DE)**

(74) Vertreter : **Huber, Bernhard, Dipl.-Chem. et al Patentanwälte H. Weickmann, Dr. K. Fincke F.A. Weickmann, B. Huber Dr. H. Liska, Dr. J. Prechtel, Dr. B. Böhm Postfach 86 08 20 D-81635 München (DE)**

EP 0 458 939 B1

## Beschreibung

Der menschliche Gewebe-Plasminogen-Aktivator (t-PA) besitzt eine große therapeutische Bedeutung bei der Auflösung von Blutgerinnseln, z.B. bei Herzinfarkten. t-PA bewirkt die Auflösung von Blutgerinnseln durch die Aktivierung von Plasminogen zu Plasmin. Plasmin wiederum löst Fibrin, die Hauptkomponente der Proteinmatrix von geronnenem Blut.

Natürlicher t-PA ist aus mehreren funktionellen Domänen F, E, K1, K2 und P zusammengesetzt. Die Domäne P beinhaltet das proteolytisch aktive Zentrum, das die Spaltung von Plasminogen zu Plasmin bewirkt. Die gentechnologische Herstellung von t-PA oder verschiedenen t-PA-Muteinen, bei denen eine oder mehrere der Domänen F, E, K1 und K2 deletiert sind, in eukaryontischen und prokaryontischen Zellen ist bereits bekannt. Dabei werden t-PA-Derivate aus Prokaryonten im Gegensatz zu natürlichem t-PA in nicht glykosylierter Form synthetisiert.

Weiterhin ist bekannt, daß der Zuckeranteil einen erheblichen Einfluß auf die Löslichkeit und Aggregation von Proteinen hat (J. Biol. Chem. 263 (1988), 8832-8837). Es wurde nun gefunden, daß nicht glykosylierter t-PA wesentlich schlechter löslich als glykosylierter t-PA ist.

In der am 22. Februar 1990 veröffentlichten W0-A-90/01 333 wird eine pharmazeutische Zusammensetzung beschrieben, die sich zur Verabreichung an einen Säuger eignet, wobei die Zusammensetzung umfaßt: ein im wesentlichen reines Gemisch von humanem Serumalbumin und t-PA mit einer spezifischen Aktivität von 50.000 IU/mg bis 400.000 IU/mg. In diesem Dokument wird nicht-glykosilierter t-PA nicht erwähnt.

Die am 9. August 1990 veröffentlichte W090/08 507 offenbart eine thrombolytische Zusammensetzung, die eine therapeutisch wirksame Menge eines verkürzten Plasminogenaktivators (ΔFE1x) in einer wäßrigen parenteralen Formulierung umfaßt, die eine solubilisierende Konzentration von Histidin und Creatinin und weiterhin gegebenenfalls eine solubilisierende Konzentration von Citrationen enthält. Auch in diesem Dokument ist keinerlei Erwähnung von nicht-glykosiliertem t-PA zu finden.

EP-A-0 297 294 beschreibt ein Verfahren zur Herstellung und Pasteurisierung einer Lösung hoher spezifischer Volumenaktivität von einem Protein mit Gewebe-Plasminogenaktivator (t-PA)-Aktivität, welches dadurch gekennzeichnet ist, daß zum Lösen mindestens zwei Substanzen aus der Gruppe der D- und/oder L-Aminosäuren, ihrer Salze, Derivate oder Homologe zugesetzt werden. In diesem Dokument findet sich keine Erwähnung von Citrat und auch keinerlei Hinweis auf ein nicht-glykosiliertes Protein mit t-PA-Aktivität.

EP-A-0 211 592 betrifft ein Verfahren zur Herstellung einer sterilen pharmazeutischen Dosierungseinheit von t-PA zur parenteralen Verabreichung, umfassend das Auflösen von t-PA in einem pharmazeutisch verträglichen, gepufferten Lösungsmittel bei pH 3,5-5,5 bis zu einer Konzentration von 0,1 - 15 mg t-PA/ml Lösung, gegebenenfalls in Gegenwart eines Gefrierschutzmittels. Als Beispiele für gepufferte Lösungsmittel werden unter anderem Adipinsäure, Glutaminsäure, Zitronensäure, Essigsäure, Bernsteinsäure, Milchsäure, Weinsäure und Asparaginsäure und Salze davon genannt. Auch in diesem Dokument findet sich keine Erwähnung, daß das dort beschriebene Präparat für nicht-glykosilierte Proteine mit t-PA-Aktivität geeignet sein könnte.

Nicht glykosylierter t-PA (t-PA pro) löst sich nur sehr schlecht in den üblicherweise zur Solubilisierung von Proteinen verwendeten Puffern, wie z.B. 50 mmol/l Na-Citrat, 50 mmol/l Phosphat oder physiologische NaCl-Lösung. Für den Einsatz als therapeutischer Wirkstoff sollte jedoch t-PA pro mit einer höheren enzymatischen Aktivität von mindestens 0,1 MU/ml, vorzugsweise 0,1 MU/ml bis 10 MU/ml vorliegen. Die Aktivität ist entsprechend WHO, National Institute for Biological Standards and Control (ZGIMAL 42 (1987), 478-486) definiert.

Aus der EP-A 0-217 379 ist bekannt, die Löslichkeit von t-PA aus Prokaryonten durch neutrale oder leicht alkalische Arginin-Formulierungen zu erhöhen. Ein Nachteil dieses Verfahrens ist jedoch, daß gute Löslichkeiten von t-PA pro nur mit sehr hohen Arginin-Konzentrationen erreicht werden können.

Aufgabe der Erfindung war es demnach, pharmazeutische Präparate zu entwickeln, die t-PA pro mit einer Aktivität von mehr als 0,1 MU/ml enthalten, wobei t-PA pro über einen längeren Zeitraum hinweg stabil sein soll.

Die erfindungsgemäße Aufgabe wird gelöst durch ein pharmazeutisches Präparat eines nicht glykosylierten Proteins mit t-PA-Aktivität mit einer enzymatischen Aktivität von mindestens 0,1 MU/ml mit einem pH von 4,5 bis 6, wobei diese Zusammensetzung Citrat und mindestens eine Verbindung aus der aus

a) Ascorbinsäure,
b) EDTA,
c) Aminoverbindungen der Formel

$$R^1R^2N - R - X$$

wobei X = $SO_3H$, $CH(NH_2)-CO_2H$, $CO_2H$, H, $NH_2$ oder OH ist, R = $C_1-C_9$-Alkylen, $C_3-C_6$-Cycloalkylen oder Benzyliden ist und $R^1$ und $R^2$ voneinander unabhängig H oder $C_1-C_3$-Alkyl sind,
d) Guanidinobuttersäure,
e) Dimethylbiguanid,

f) Arginin,

h) Pyrimidinnukleosiden und Pyrimidinnukleotiden,

i) mit einer oder mehreren Hydroxy-, Keto- oder/und weiteren Carboxygruppen substituierte Carbonsäuren bestehenden Gruppe enthält.

Unter t-PA pro gemäß vorliegender Erfindung versteht man einen t-PA, der mit den Aminosäuren -3 (Gly) bis +1 (Ser) beginnt und bei 527 (Pro) endet (Nomenklatur nach Harris, Protein Engineering, Band 1 (1987) 449-458). Ein t-PA pro ist nach dem in der WO 87/02673 beschriebenen Verfahren erhältlich.

Für die Solubilisierung von t-PA pro ist ein Citrat-Puffer besonders geeignet. Die Konzentration der Citrationen soll mindestens 5 mmol/l, vorzugsweise von 5 bis 100 mmol/l betragen, besonders bevorzugt ist eine Konzentration der Citrationen von 50 mmol/l. Der pH-Wert wird je nach Basizität der zugesetzten Verbindung vorzugsweise mit HCl oder einer Base wie z.B. NaOH oder KOH eingestellt.

Überraschenderweise wurde festgestellt, daß die Löslichkeit von nicht glykosyliertem t-PA in anderen Puffersystemen, z.B. Phosphatpuffer, bei gleicher Ionenstärke und gleichem pH-Wert wesentlich geringer ist. Es hat sich als geeignet erwiesen, den pH-Wert von alkalischen Citratlösungen mit HCl einzustellen, d.h. daß die Zusammensetzung zusätzlich noch Chloridionen enthält. In Gegenwart von Chloridionen sind nämlich überraschenderweise hochkonzentrierte Lösungen von t-PA pro wesentlich stabiler als z.B. in Gegenwart von Phosphationen. Der pH-Wert von sauren Citratlösungen wird üblicherweise mit NaOH eingestellt.

Geeignet für eine erfindungsgemäße Zusammensetzung ist ein pH-Wert zwischen 4,5 und 6,5, bevorzugt ist ein pH-Wert von 6.

Vorzugsweise werden für eine erfindungsgemäße Zusammensetzung als Aminoverbindungen Taurin, 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Aminobutan, 1,3-Aminopropan, Lysin, Ornithin, 8-Aminooctansäure, 7-Aminoheptansäure, ε-Aminocapronsäure, δ-Aminovaleriansäure, γ-Aminobuttersäure, Tranexamsäure oder p-Aminomethylbenzoesäure verwendet. Die bevorzugte Konzentration für Taurin und analoge Verbindungen beträgt 0,1 bis 0,5 mol/l, besonders bevorzugt 0,1 bis 0,3 mol/l. 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Diaminobutan oder 1,3-Diaminopropan werden vorzugsweise mit 10 bis 100 mmol/l eingesetzt. Lysin, Ornithin, 8-Aminooctansäure, 7-Aminoheptansäure, ε-Aminocapronsäure, δ-Aminovaleriansäure, γ-Aminobuttersäure, Tranexamsäure oder p-Aminomethylbenzoesäure werden vorzugsweise mit 0,5 bis 20 mmol/l, besonders bevorzugt mit 1 bis 10 mmol/l verwendet.

Als Carbonsäure, die mit einer oder mehreren Hydroxy-, Keto- oder/und weiteren Carboxygruppen substituiert ist, verwendet man z.B. Apfelsäure, Milchsäure, Fumarsäure oder Oxoglutarsäure. Diese Substanzen werden vorzugsweise mit 1 mmol/l bis 1000 mmol/l, besonders bevorzugt mit 10 bis 500 mmol/l verwendet.

Guanidinobuttersäure oder Arginin werden vorzugsweise mit 10 bis 200 mmol/l, besonders bevorzugt mit 50 bis 100 mmol/l verwendet. Für Dimethylbiguanid beträgt die Konzentration 50 bis 400 mmol/l, vorzugsweise 100 bis 300 mmol/l.

EDTA wird vorzugsweise mit 1 bis 100 mmol/l, besonders bevorzugt mit 10 bis 100 mmol/l verwendet. Ascorbinsäure wird vorzugsweise mit 0,1 bis 1 mol/l, besonders bevorzugt mit 0,2 bis 0,3 mol/l eingesetzt.

Als Pyrimidinnukleoside und Pyrimidinnukleotide sind z.B. Thymidin, Cytosin und Uridin bzw. die entsprechenden Nukleotide geeignet. Sie werden vorzugsweise in Konzentrationen von 1 bis 300 mmol/l, besonders bevorzugt 10 bis 300 mmol/l eingesetzt.

Weiterhin ein Gegenstand der Erfindung ist eine erfindungsgemäße Zusammensetzung, die zusätzlich eine oder mehrere Aminosäuren, insbesondere Histidin enthält.

Im folgenden ist eine Reihe besonders bevorzugter Präparate gemäß vorliegender Erfindung aufgeführt.

Eine Formulierung enthält 50 mmol/l Na-Citrat/NaOH, pH 6 und 0,1 bis 0,3 mol/l Taurin. Bevorzugt ist auch eine Formulierung mit 50 mmol/l Na-Citrat, pH 6 und 0,2 bis 0,3 mol/l Ascorbinsäure.

Weiterhin bevorzugt ist eine Formulierung mit 50 mmol/l Na-Citrat/HCl, pH 6 und 1 mmol/l bis 10 mmol/l 7-Aminoheptansäure, 8-Aminooctansäure, p-Aminomethylbenzoesäure, ε-Aminocapronsäure, δ-Aminovaleriansäure, γ-Aminobuttersäure, Tranexamsäure, Lysin oder Ornithin.

Weiterhin besonders bevorzugt sind auch Formulierungen, die 50 mmol/l Na-Citrat/HCl, pH 6,0 und 50 bis 100 mmol/l Guanidinobuttersäure oder Arginin enthalten.

Bevorzugt ist auch eine Formulierung, die 50 mmol/l Na-Citrat, pH 6 und 10 bis 100 mmol/l EDTA enthält. Wiederum eine weitere Formulierung enthält 50 mmol/l NaCitrat/HCl, pH 6 und 100 bis 300 mmol/l Dimethylbiguanid.

Eine weitere Formulierung enthält 50 mmol/l NaCitrat/HCl, pH 6 und 10 bis 300 mmol/l Thymidin, Cytosin oder Uridin. Wiederum eine weitere Formulierung enthält 50 mmol/l NaCitrat/HCl, pH 6 und 10 bis 100 mmol/l 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Diaminobutan oder 1,3-Diaminopropan.

Schließlich enthält eine weitere Formulierung 50 mmol/l NaCitrat, pH 6 und 10 bis 500 mmol/l Apfelsäure, Milchsäure, Fumarsäure oder 2-Oxoglutarsäure.

Auch Kombinationen aus mehreren der obengenannten Verbindungen mit Citrat bewirken eine sehr gute Löslichkeit von Proteinen mit t-PA-Aktivität.

Ein Gegenstand der Erfindung ist schließlich auch ein Arzneimittel auf Basis eines nicht-glykosilierten Proteins mit t-PA-Aktivität als Wirkstoff in Lösung oder als Lyophilisat mit den angegebenen Wirkstoffen und gegebenenfalls noch weiteren Pharmazeutisch verträglichen Zusatz-, Hilfs-, Träger- und Füllstoffen.

Die erfindungsgemäßen pharmazeutischen Präparate kommen vorzugsweise als Injektions- und Infusionslösungen zum Einsatz. Dies kann dadurch geschehen, daß eine bereits spritzfertige Lösung zur Verfügung gestellt wird, die die erfindungsgemäße Zusammensetzung besitzt. Es ist jedoch auch möglich, die pharmazeutischen Präparate in Form von Lyophilisaten zur Verfügung zu stellen. Diese werden dann mit an sich bekannten, zu Injektionszwecken geeigneten Mitteln oder Lösungen rekonstituiert. Als Injektionsmedium kommt vorzugsweise Wasser zur Anwendung, welches die bei Injektionslösungen üblichen Zusätze wie Stabilisierungsmittel, Lösungsvermittler, Puffer und isotonische Zusätze, beispielsweise eine physiologische NaCl-Konzentration, enthält. Derartige Zusätze sind beispielsweise Mannit, Tartrat- oder Citrat-Puffer, Ethanol, Komplexbildner wie z.B. Ethylendiamintetraessigsäure und deren nicht-toxischen Salze, sowie hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt.

Schließlich beinhaltet die Erfindung auch die Verwendung von t-PA pro zur Herstellung erfindungsgemäßer pharmazeutischer Präparate.

Die folgenden Beispiele sollen die konkrete Ausführung der Erfindung weiter erläutern.

**Beispiel 1**

**Löslichkeit von t-PA pro**

Gereinigtes t-PA pro (gelöst in 0.5 mol/l Arginin/$H_3PO_4$, pH 7,2) wird durch Ultrafiltration über eine YM 10-Membran (Amicon) konzentriert. Jeweils 1 ml des Konzentrats (Aktivität: 2.4 MU/ml) wird gegen die in Tabelle 1 aufgeführten Puffer dialysiert. Nach Zentrifugation der Proben wird im klaren Überstand die enzymatische Aktivität gemessen.

Die enzymatische Aktivität ist als Volumeneinheit in MU/ml und als Gesamtaktivität in MU angegeben.

Die Messung der tPA-Aktivität kann dabei auf übliche Weise durch Spaltung eines chromogenen Substrats bestimmt werden (H. Lill, ZGIMAL 42 (1987), 478 - 486). Die Einheit U ist eine Einheit der Aktivität nach Definition der WHO, National Institute for Biological Standards and Control.

## T a b e l l e 1

| Puffer | Aktivität | |
| --- | --- | --- |
| | MU/ml | MU |
| 50 mmol/l NaCitrat/NaOH, pH 6<br>0,3 mol/l Taurin | 0,34 | 0,20 |
| 50 mmol/l NaCitrat/NaOH, pH 6<br>0,3 mol/l Ascorbinsäure | 0,34 | 0,18 |
| 50 mmol/l NaCitrat/HCl, pH 6<br>10 mmol/l ∈-Aminocapronsäure | 0,21 | 0,28 |

| Puffer | Aktivität | |
|---|---|---|
| | MU/ml | MU |
| 50 mmol/l NaCitrat/HCl, pH 6 10 mmol/l Lysin | 0,17 | 0,12 |
| 50 mmol/l NaCitrat/HCl, pH 6 10 mmol/l Tranexamsäure | 0,26 | 0,36 |
| 50 mmol/l NaCitrat/HCl, pH 6 10 mmol/l p-Aminomethyl-benzoesäure | 0,24 | 0,33 |
| 50 mmol/l NaCitrat/HCl, pH 6 0,3 mol/l Dimethylbiguanid | 1,20 | 1,50 |
| 0,05 mol/l Tris/HCl, pH 7,2 | 0,01 | 0,01 |
| 50 mmol/l $NH_4HCO_3$ | 0,05 | 0,04 |
| 50 mmol/l NaCitrat/HCl, pH 6 10 mmol/l δ-Aminovalerian-säure | 0,17 | 0,23 |
| 50 mmol/l NaCitrat/HCl, pH 6 10 mmol/l γ-Aminobuttersäure | 0,27 | 0,20 |
| 50 mmol/l NaCitrat/HCl, pH 6 10 mmol/l 7-Aminoheptansäure | 0,20 | 0,27 |
| 50 mmol/l NaCitrat/HCl, pH 6 50 mmol/l Arginin | 0,19 | 0,21 |

| Puffer | Aktivität | |
|---|---|---|
| | MU/ml | MU |
| 50 mmol/l NaCitrat/HCl, pH 6<br>50 mmol/l Guanidinobutter-<br>säure | 0,16 | 0,19 |
| 50 mmol/l NaCitrat/HCl, pH 6<br>10 mmol/l EDTA | 0,10 | 0,12 |
| 50 mmol/l NaCitrat/NaOH, pH 6<br>50 mmol/l EDTA | 0,18 | 0,22 |
| 50 mmol/l NaCitrat/NaOH, pH 6<br>100 mmol/l EDTA | 0,26 | 0,29 |
| 50 mmol/l NaCitrat/HCl, pH 6<br>50 mmol/l 1,6 Diaminohexan | 0,29 | 0,37 |
| 50 mmol/l NaCitrat/HCl, pH 6<br>50 mmol/l 5-Aminopentanol | 0,29 | 0,36 |
| 50 mmol/l NaCitrat/HCl, pH 6<br>0,1 mol/l Thymidin | 0,16 | 0,21 |
| 50 mmol/l NaCitrat/HCl, pH 6 | 0,07 | 0,09 |
| 50 mmol/l $Na_2HPO_4/H_3PO_4$, pH 6 | 0,02 | 0,03 |
| 50 mmol/l NaCitrat/NaOH, pH 6<br>0,3 mol/l Fumarsäure | 0,19 | 0,19 |

**Patentansprüche**

1.  Pharmazeutisches Präparat eines nicht glykosylierten Proteins mit t-PA-Aktivität mit einer enzymatischen Aktivität von mindestens 0,1 MU/ml und einem pH-Wert von 4,5 bis 6,

**dadurch gekennzeichnet,**

daß es Citrat und mindestens eine Verbindung aus der aus

    a) Ascorbinsäure,

    b) EDTA,

    c) Aminoverbindungen der Formel

$$R^1R^2N - R - X$$

    wobei X = $SO_3H$, $CH(NH_2)$-$CO_2H$, $CO_2H$, H, $NH_2$ oder OH ist, R = $C_1$-$C_9$-Alkylen, $C_3$-$C_6$-Cycloalkylen
    oder Benzyliden ist und $R^1$ und $R^2$ voneinander unabhängig H oder $C_1$-$C_3$-Alkyl sind,

    d) Guanidinobuttersäure,

    e) Dimethylbiguanid,

    f) Arginin,

    h) Pyrimidinnukleosiden und Pyrimidinnukleotiden,

    i) mit einer oder mehreren Hydroxy-, Keto- oder/und weiteren Carboxygruppen substituierte Carbonsäuren

bestehenden Gruppe enthält.

2.   Pharmazeutisches Präparat nach Anspruch 1,
    **dadurch gekennzeichnet,**

    daß die Aminoverbindung Taurin, 4-Aminobutanol-1, 5-Aminopentanol-1, 6-Aminohexanol-1, 1,9-Diaminononan, 1,8-Diaminooctan, 1,7-Diaminoheptan, 1,6-Diaminohexan, 1,5-Diaminopentan, 1,4-Aminobutan, 1,3-Aminopropan, Lysin, Ornithin, 8-Aminooctansäure, 7-Aminoheptansäure, ε-Aminocapronsäure,
    δ-Aminovaleriansäure, γ-Aminobuttersäure, Tranexamsäure oder p-Aminomethylbenzoesäure ist.

3.   Pharmazeutisches Präparat nach Anspruch 1,
    **dadurch gekennzeichnet,**

    daß die mit einer oder mehreren Hydroxy-, Keto- oder/und weiteren Carboxygruppen substituierte Carbonsäure Apfelsäure, Milchsäure, Fumarsäure oder 2-Oxoglutarsäure ist.

4.   Pharmazeutisches Präparat nach Anspruch 1,
    **dadurch gekennzeichnet,**

    daß es zusätzlich eine oder mehrere Aminosäuren enthält.

5.   Pharmazeutisches Präparat nach einem der Ansprüche 1 bis 4,
    **dadurch gekennzeichnet,**

    daß die Citrat-Konzentration 5 bis 100 mmol/l, vorzugsweise 50 mmol/l beträgt.

6.   Pharmazeutisches Präparat nach einem der Ansprüche 1 bis 5,
    **dadurch gekennzeichnet,**

    daß es zusätzlich Chlorid-Ionen enthält.

7.   Pharmazeutisches Präparat nach Anspruch 1,
    **dadurch gekennzeichnet,**

    daß es 50 mmol/l NaCitrat, pH 6, und 0,1 bis 1 mol/l, vorzugsweise 0,2 bis 0,3 mol/l Ascorbinsäure enthält.

8.   Pharmazeutisches Präparat nach Anspruch 1,
    **dadurch gekennzeichnet,**

    daß es 50 mmol/l NaCitrat, pH 6, und 1 bis 200 mmol/l, vorzugsweise 10 bis 100 mmol/l EDTA enthält.

9.   Pharmazeutisches Präparat nach Anspruch 1,
    **dadurch gekennzeichnet,**

    daß es 50 mmol/l NaCitrat, pH 6, und 0,1 bis 0,5 mol/l, vorzugsweise 0,1 bis 0,3 mol/l Taurin enthält.

10.  Pharmazeutisches Präparat nach Anspruch 1,
    **dadurch gekennzeichnet,**

    daß es 50 mmol/l NaCitrat/HCl, pH 6 und 0,5 bis 20 mmol/l Lysin, Ornithin, 8-Aminooctansäure, 7-Aminoheptansäure, ε-Aminocapronsäure, δ-Aminovaleriansäure, γ-Aminobuttersäure, Tranexamsäure oder
    p-Aminomethylbenzoesäure enthält.

11.  Pharmazeutisches Präparat nach Anspruch 1,

**dadurch gekennzeichnet,**
daß es 50 mmol/l NaCitrat/HCl, pH 6, und 10 bis 100 mmol/l 4-Aminobutanol-1, 5-Aminopentanol-1, 6-Aminohexanol-1, 1,3-Diaminopropan, 1,4-Diaminobutan, 1,5-Diaminopentan, 1 6-Diaminohexan, 1,7-Diaminoheptan, 1,8-Diaminooctan oder 1,9-Diaminononan enthält.

12. Pharmazeutisches Präparat nach Anspruch 1,
    **dadurch gekennzeichnet,**
    daß es 50 mmol/l NaCitrat/HCl, pH 6, und 10 bis 200 mmol/l, vorzugsweise 50 bis 100 mmol/l Guanidinobuttersäure oder Arginin enthält.

13. Pharmazeutisches Präparat nach Anspruch 1,
    **dadurch gekennzeichnet,**
    daß es 50 mmol/l NaCitrat/HCl und 50 bis 400 mmol/l, vorzugsweise 100 bis 300 mmol/l Dimethylbiguanid enthält.

14. Pharmazeutisches Präparat nach Anspruch 1,
    **dadurch gekennzeichnet,**
    daß es 50 mmol/l NaCitrat/HCl, pH 6, und 1 bis 300 mmol/l, vorzugsweise 10 bis 300 mmol/l Thymidin, Cytosin oder Uridin enthält.

15. Pharmazeutisches Präparat nach Anspruch 1,
    **dadurch gekennzeichnet,**
    daß es 50 mmol/l NaCitrat, pH 6, und 0,001 bis 1 mol/l, vorzugsweise 0,01 bis 0,5 mol/l Apfelsäure, Milchsäure, Fumarsäure oder 2-Oxoglutarsäure enthält.

16. Pharmazeutisches Präparat nach Anspruch 1,
    **dadurch gekennzeichnet,**
    daß es 50 mmol/l NaCitrat, pH 6, und eine Kombination von Substanzen aus der Gruppe a) bis i) nach Anspruch 1 enthält.

17. Arzneimittel auf Basis eines nicht-glykosilierten Proteins mit t-PA-Aktivität als Wirkstoff,
    **gekennzeichnet durch**
    die Zusammensetzung nach einem der vohergehenden Ansprüche, gegebenenfalls zusammen mit üblichen pharmazeutischen Zusatz-, Hilfs- oder/und Trägerstoffen.

18. Verfahren zur Herstellung eines pharmazeutischen Präparats nach einem der Ansprüche 1 bis 17,
    **dadurch gekennzeichnet,**
    daß man nicht glykosylierten t-PA zusammen mit mindestens einer Substanz aus der Gruppe a) bis i) nach Anspruch 1 in eine geeignete pharmazeutische Darreichungsform überführt.

19. Verfahren nach Anspruch 18,
    **dadurch gekennzeichnet,**
    daß die pharmazeutische Darreichungsform eine Injektionslösung oder ein Lyophilisat ist.

20. Verwendung von nicht glykosyliertem t-PA zur Herstellung von pharmazeutischen Präparaten nach einem der Ansprüche 1 bis 17.

**Claims**

1. Pharmaceutical preparation of a non-glycosylated protein with t-PA activity with an enzymatic activity of at least 0.1 MU/ml and a pH value of 4.5 to 6, characterised in that it contains citrate and at least one compound from the group consisting of
   a) ascorbic acid,
   b) EDTA,
   c) amino compounds of the formula

   $$R^1R^2N - R - X$$

   whereby X = $SO_3H$, $CH(NH_2)-CO_2H$, $CO_2H$, H, $NH_2$ or OH, R = $C_1$-$C_9$-alkylene, $C_3$-$C_6$-cycloalkylene or benzylidene and $R^1$ and $R^2$, independently of one another, are H or $C_1$-$C_3$-alkyl,

d) guanidinobutyric acid,

e) dimethylbiguanide,

f) arginine,

h) pyrimidine nucleosides and pyrimidine nucleotides,

i) carboxylic acids substituted with one or more hydroxyl, keto and/or further carboxyl groups.

2. Pharmaceutical preparation according to claim 1, characterised in that the amino compound is taurine, 4-aminobutanol-1, 5-aminopentanol-1, 6-aminohexanol-1, 1,9-diaminononane, 1,8-diaminooctane, 1,7-diaminoheptane, 1,6-diaminohexane, 1,5-diaminopentane, 1,4-aminobutane, 1,3-aminopropane, lysine, ornithine, 8-aminooctanoic acid, 7-aminoheptanoic acid, ε-aminocaproic acid, δ-sminovaleric acid, γ-aminobutyric acid, tranexamic acid or p-aminomethylbenzoic acid.

3. Pharmaceutical preparation according to claim 1, characterised in that the carboxylic acid substitited with one or more hydroxyl, keto and/or further carboxyl groups is malic acid, lactic acid, fumaric acid or 2-oxoglutaric acid.

4. Pharmaceurical preparation according to claim 1, characterised in that it additionally contains one or more amino acids.

5. Pharmaceutical preparation according to one of claims 1 to 4, characterised in that the citrate concentration amounts to 5 to 100 mmol/l, preferably 50 mmol/l.

6. Pharmaceutical preparation according to one of claims 1 to 5, characterised in that it additionally contains chloride ions.

7. Pharmaceutical preparation according to claim 1, characterised in that it contains 50 mmol/l Na citrate, pH 6, and 0.1 to 1 mol/l, preferably 0.2 to 0.3 mol/l ascorbic acid.

8. Pharmaceutical preparation according to claim 1, characterised in that it contains 50 mmol/l Na citrate, pH 6, and 1 to 200 mmol/l, preferably 10 to 100 mmol/l EDTA.

9. Pharmaceutical preparation according to claim 1, characterised in that it contains 50 mmol/l Na citrate, pH 6, and 0.1 to 0.5 mol/l, preferably 0.1 to 0.3 mol/l taurine.

10. Pharmaceutical preparation according to claim 1, characterised in that it contains 50 mmol/l Na citrate/HCl, pH 6, and 0.5 to 20 mmol/l lysine, ornithine, 8-aminooctanoic acid, 7-aminoheptanoic acid, ε-aminocaproic acid, δ-aminovaleric acid, γ-aminobutyric acid, tranexamic acid or p-aminomethylbenzoic acid.

11. Pharmaceutical preparation according to claim 1, characterised in that it contains 50 mmol/l Na citrate/HCl, pH 6, and 10 to 100 mmol/l 4-aminobutanol-1, 5-aminopentanol-1, 6-aminohexanol-1, 1,3-diaminopropane, 1,4-diaminebutane, 1,5-diaminopentane, 1,6-diaminohexane, 1,7-diaminoheptane, 1,8-diaminooctane or 1,9-diaminononane.

12. Pharmaceutical preparation according to claim 1, characterised in that it contains 50 mmol/l Na citrate/HCl, pH 6, and 10 to 200 mmol/l, preferably 50 to 100 mmol/l guanidinobutyric acid or arginine.

13. Pharmaceutical preparation according to claim 1, characterised in that it contains 50 mmol/l Na citrate/HCl, and 50 to 400 mmol/l, preferably 100 to 300 mmol/l dimethylbiguanide.

14. Pharmaceutical preparation according to claim 1, characterised in that it contains 50 mmol/l Na citrate/HCl, pH 6, and 1 to 300 mmol/l, preferably 10 to 300 mmol/l thymidine, cytosine or uridine.

15. Pharmaceutical preparation according to claim 1, characterised in that it contains 50 mmol/l Na citrate, pH 6, and 0.001 to 1 mol/l, preferably 0.01 to 0.5 mol/l malic acid, lactic acid, fumaric acid or 2-oxoglutaric acid.

16. Pharmaceutical preparation according to claim 1, characterised in that it contains 50 mmol/l Na citrate, pH 6, and a combination of substances from the group a) to i) according to claim 1.

17. Medicament based on a non-glycosylated protein with t-PA activity, characterised by a composition according to one of the preceding claims, possibly together with usual pharmaceutical additive, adjuvant and/or carrier materials.

18. Process for the production of a pharmaceutical preparation according to one of claims 1 to 17, characterised in that one converts non-glycosylated t-PA, together with at least one substance of the group a) to i) according to claim 1, into a suitable pharmaceutical form of administration.

19. Process according to claim 18, characterised in that the pharmaceutical form of administration is an injection solution or a lyophilisate.

20. Use of non-glgcosylated t-PA for the production of pharmaceutical preparations according to one of claims 1 to 17.

**Revendications**

1. Préparation pharmaceutique d'une protéine non glycosylée à activité du t-PA présentant une activité enzymatique d'au moins 0,1 mU/ml et un pH de 4,5 à 6, caractérisée en ce qu'elle contient un citrate et au moins un composé du groupe formé par
    a) l'acide ascorbique,
    b) EDTA,
    c) des composés aminés de formule
$$R^1R^2N - R - X$$
    où X représente $SO_3H$, $CH(NH_2)$-$CO_2H$, $CO_2H$, H, $NH_2$ ou OH, R représente un groupe alkylène en $C_1$-$C_9$, cycloalkylène en $C_3$-$C_6$ ou benzylidène et $R^1$ et $R^2$ représentent indépendamment l'un de l'autre H ou un groupe alkyle en $C_1$-$C_3$,
    d) l'acide guanidinobutyrique,
    e) le diméthylbiguanide,
    f) l'arginine,
    h) les nucléosides pyrimidiques et les nucléotides pyrimidiques,
    i) les acides carboxyliques substitués par un ou plusieurs groupes hydroxyle, cétone et/ou carboxyle supplémentaires.

2. Préparation pharmaceutique selon la revendication 1, caractérisée en ce que le composé aminé est la taurine, le 4-aminobutan-1-ol, le 5-aminopentan-1-ol, le 6-aminohexan-1-ol, le 1,9-diaminononane, le 1,8-diaminooctane, le 1,7-diaminoheptane, le 1,6-diaminohexane, le 1,5-diaminopentane, le 1,4-diaminobutane, le 1,3-diaminopropane, la lysine, l'ornithine, l'acide 8-aminooctanoïque, l'acide 7-aminoheptanoïque, l'acide $\varepsilon$-aminocaproïque, l'acide $\delta$-aminovalérique, l'acide $\gamma$-aminobutyrique, l'acide tranexamique ou l'acide p-aminométhylbenzoïque.

3. Préparation pharmaceutique selon la revendication 1, caractérisée en ce que l'acide carboxylique substitué par un ou plusieurs groupes hydroxyle, cétone et/ou carboxyle supplémentaires est l'acide malique, l'acide lactique, l'acide fumarique ou l'acide 2-oxoglutarique.

4. Préparation pharmaceutique selon la revendication 1, caractérisée en ce qu'elle contient en outre un ou plusieurs acides aminés.

5. Préparation pharmaceutique selon l'une des revendications 1 à 4, caractérisée en ce que la concentration du citrate est de 5 à 100 mmol/l, de préférence de 50 mmol/l.

6. Préparation pharmaceutique selon l'une des revendications 1 à 5, caractérisée en ce qu'elle contient en outre des ions chlorure.

7. Préparation pharmaceutique selon la revendication 1, caractérisée en ce qu'elle contient 50 mmol/l de citrate de Na, pH 6, et 0,1 à 1 mol/l, de préférence 0,2 à 0,3 mol/l d'acide ascorbique.

8. Préparation pharmaceutique selon la revendication 1, caractérisée en ce qu'elle contient 50 mmol/l de citrate de Na, pH 6, et 1 à 200 mmol/l, de préférence 10 à 100 mmol/l d'EDTA.

9. Préparation pharmaceutique selon la revendication 1, caractérisée en ce qu'elle contient 50 mmol/l de citrate de Na, pH 6, et 0,1 à 0,5 mol/l, de préférence 0,1 à 0,3 mol/l de taurine.

10. Préparation pharmaceutique selon la revendication 1, caractérisée en ce qu'elle contient 50 mmol/l de citrate de Na/HCl, pH 6, et 0,5 à 20 mmol/l de lysine, d'ornithine, d'acide 8-aminooctanoïque, d'acide 7-aminoheptanoïque, d'acide ε-aminocaproïque, d'acide δ-aminovalérique, d'acide y-aminobutyrique, d'acide tranexamique ou d'acide p-aminométhylbenzoïque.

11. Préparation pharmaceutique selon la revendication 1, caractérisée en ce qu'elle contient 50 mmol/l de citrate de Na/HCl, pH 6, et 10 à 100 mmol/l de 4-aminobutan-1-ol, de 5-aminopentan-1-ol, de 6-aminohexan-1-ol, de 1,3-diaminopropane, de 1,4-diaminobutane, de 1,5-diaminopentane, de 1,6-diaminohexane, de 1,7-diaminoheptane, de 1,8-diaminooctane ou de 1,9-diaminononane.

12. Préparation pharmaceutique selon la revendication 1, caractérisée en ce qu'elle contient 50 mmol/l de citrate de Na/HCl, pH 6, et 10 à 200 mmol/l, de préférence 50 à 100 mmol/l d'acide guanidinobutyrique ou d'arginine.

13. Préparation pharmaceutique selon la revendication 1, caractérisée en ce qu'elle contient 50 mmol/l de citrate de Na/HCl et 50 à 400 mmol/l, de préférence 100 à 300 mmol/l de diméthylbiguanide.

14. Préparation pharmaceutique selon la revendication 1, caractérisée en ce qu'elle contient 50 mmol/l de citrate de Na/HCl, pH 6, et 1 à 300 mmol/l, de préférence 10 à 300 mmol/l de thymidine, de cytosine ou d'uridine.

15. Préparation pharmaceutique selon la revendication 1, caractérisée en ce qu'elle contient 50 mmol/l de citrate de Na, pH 6, et 0,001 à 1 mol/l, de préférence 0,01 à 0,5 mol/l d'acide malique, d'acide lactique, d'acide fumarique ou d'acide 2-oxoglutarique.

16. Préparation pharmaceutique selon la revendication 1, caractérisée en ce qu'elle contient 50 mmol/l de citrate de Na, pH 6, et une combinaison de substances du groupe a) à i) selon la revendication 1.

17. Médicament à base d'une protéine non glycosylée à activité du t-PA en tant que principe actif, caractérisé par la composition selon l'une des revendications précédentes, éventuellement avec des additifs, adjuvants et/ou véhicules pharmaceutiques courants.

18. Procédé d'obtention d'une préparation pharmaceutique selon l'une des revendications 1 à 17, caractérisé en ce que l'on convertit du t-PA non glycosylé et au moins une substance du groupe a) à i) selon la revendication 1 en une forme d'administration pharmaceutique appropriée.

19. Procédé selon la revendication 18, caractérisé en ce que la forme d'administration pharmaceutique est une solution pour injection ou un lyophilisat.

20. Utilisation de t-PA non glycosylé pour l'obtention de préparations pharmaceutiques selon l'une des revendications 1 à 17.